# EUROPÄISCHE PATENTSCHRIFT

(11) **EP 1 303 219 B1**
(45) Veröffentlichungstag und Bekanntmachung des Hinweises auf die Patenterteilung: **06.09.2006**
(21) Anmeldenummer: 01947436.0
(22) Anmeldetag: 03.07.2001
(51) Int. Cl.: A61B 17/32

(54) **MEDIZINISCHES INSTRUMENT, INSBESONDERE RESEKTOSKOP**
MEDICAL INSTRUMENT, ESPECIALLY A RESECTOSCOPE
INSTRUMENT MEDICAL, NOTAMMENT RESECTOSCOPE

(30) Priorität: 22.07.2000 DE 10035722
(43) Veröffentlichungstag der Anmeldung: 23.04.2003
(73) Patentinhaber: Karl Storz GmbH & Co. KG, 78532 Tuttlingen (DE)
(72) Erfinder: BERBERICH, Sascha, 78532 Tuttlingen (DE); DOLL, Frank, 78589 Dürbheim (DE)
(74) Vertreter: Hofmeister, Frank
(86) Internationale Anmeldenummer: PCT/EP2001/007580
(87) Internationale Veröffentlichungsnummer: WO 2002/007616

(56) Entgegenhaltungen:
- DE-A- 3 917 583

## Beschreibung

Die Erfindung betrifft ein medizinisches Instrument, insbesondere Resektoskop, mit einer Handhabe, an der an einem Handhabenteil der Handhabe drehbar gelagert mindestens ein Griffteil, insbesondere ein Fingerring, über eine Rastverbindung lösbar festgelegt ist, wobei die Rastverbindung aus einem an einem der miteinander zu verbindenden Bauteile ausgebildeten Schaft und einer am jeweils anderen Bauteil ausgebildeten Hülse zur Aufnahme des Schaftes besteht und der Außendurchmesser des Schaftes im Bereich des freien Endes des Schaftes eine Hinterschneidung bildend den Kerndurchmesser des Schaftes übersteigt und der Innendurchmesser der Hülse entsprechend einen eine Hinterschneidung bildenden und zur Aufnahme des freien Endes des Schaftes dienenden Bereich mit einem größeren Innendurchmesser und einen sich in Richtung der Hülsenöffnung erstreckenden Bereich mit einem geringeren Innendurchmesser aufweist.

Resektoskope werden in der Chirurgie verwendet, um unter endoskopischer Sicht Gewebe- und/oder Organteile zu entfernen. Zur Betätigung des in einem hohlen Schaft verschiebbar gelagerten Werkzeugs weist die Handhabe der Resektoskope einen starren und einen beweglichen Handhabenteil auf. Um das Instrument sicher und präzise führen zu können, ist meist am proximalen Handhabenteil ein Griffteil in Form eines Fingerrings angeordnet, über den der Operateur den Handhabenteil und somit auch das Werkzeug exakt führen kann. Die Bewegungsfreiheit des Operateurs wird dabei dadurch erhöht, daß der Fingerring in der Regel um die Längsachse frei drehbar an dem einen Handhabenteil angeordnet ist.

Aus der Praxis sind Resektoskope bekannt, bei denen der Fingerring lösbar an einem Handhabenteil festgelegt ist, um die Fingerringe in Größe und Form an den jeweiligen Operateur anpassen zu können, ohne dafür ein separates Resektoskop vorrätig halten zu müssen. Zu diesem Zweck ist der Fingerring dieser bekannten Resektoskope um seine Längsrichtung drehbar an einer Platte gelagert, die ihrerseits mit dem beweglichen Handhabenteil der Handhabe verschraubbar ist. Das Wechseln des Fingerrings erfolgt somit durch Abschrauben und Austauschen der den Fingerring tragenden Platte. Trotz der vielseitigeren Verwendbarkeit dieser Resektoskope weisen diese bekannten medizinischen Instrumente den Nachteil auf, daß die Teile zum lösbaren Festlegen des Fingerrings an dem einen Handhabenteil aus mehreren spanend zu fertigenden Teilen bestehen, weshalb diese Ausgestaltung sehr aufwendig und teuer ist.

Ein gattungsgemäßes medizinisches Instrument ist schließlich aus DE 39 17 583 C2 bekannt. Bei diesem bekannten, als Resektoskop ausgebildeten medizinischen Instrument, ist die Rastverbindung zum Festlegen des mindestens einen drehbaren Griffteils an der Handhabe so ausgebildet, daß der mit dem Griffteil verbundene Schaft an seinem freien Ende eine Hinterschneidung bildend den Kerndurchmesser des Schaftes übersteigt und die in der Handhabe ausgebildete Hülse zur Aufnahme dieses Schaftes einen zur Aufnahme des freien Endes des Schaftes dienenden Bereich mit einem größeren Innendurchmesser aufweist. Das freie Ende des über einen in Längsrichtung des Schaftes verlaufenden Schlitz verformbaren Schaftes wird beim Einschieben in die Hülse zunächst zusammengepreßt, bevor es sich in dem Bereich der Hülse mit dem größerem Innendurchmesser wieder aufweiten kann. Dieses Aufweiten bewirkt ein Hintergreifen der am Schaft ausgebildeten Hinterschneidung und somit ein verrastendes Festlegen des Schaftes in der Hülse.

Zwar ermöglicht diese bekannte Rastverbindung bei kostengünstiger Herstellung ein schnelles und einfaches Festlegen des Griffteils an der Handhabe, jedoch ist der Griffteil auch ebenso einfach durch bloßes Aufbringen einer axialen Zugkraft auf den Griffteil wieder von der Handhabe lösbar, so daß es insbesondere nach einem häufigen Gebrauch der Rastverbindung zu einer unbeabsichtigten Entrastung während des operativen Gebrauchs des medizinischen Instruments kommen kann, wenn die Rastverbindung weich geworden ist.

Davon ausgehend liegt der Erfindung die **Aufgabe** zugrunde, ein medizinisches Instrument der eingangs genannten Art so weiterzuentwickeln, daß der Griffteil einerseits leicht montierbar und demontierbar an der Handhabe gelagert ist sowie fertigungstechnisch einfach und kostengünstig herstellbar ist, andererseits aber zusätzlich eine sichere Festlegung an der Handhabe gewährleistet.

Die **Lösung** dieser Aufgabenstellung ist erfindungsgemäß dadurch gekennzeichnet, daß der Schaft im Bereich des den größeren Außendurchmesser aufweisenden freien Endes mit einem Außengewinde versehen ist und in der Hülse im Bereich des geringsten Innendurchmessers ein Innengewinde ausgebildet ist.

Durch die erfindungsgemäße Anordnung einer Gewindestrecke in der Rastverbindung werden zwar die Montage und Demontage geringfügig erschwert, das unbeabsichtigte Lösen des Griffteils von der Handhabe aufgrund einer bloßen axialen Zugkraft, wird durch diese Ausgestaltung aber vollständig verhindert, wodurch die Gebrauchstauglichkeit eines solchermaßen ausgebildeten medizinischen Instruments deutlich verbessert wird.

Der Griffteil kann direkt mit einem Handhabenteil oder indirekt über ein an dem Handhabenteil festgelegtes Anschlußstück mit dem Handhabenteil verbunden sein, wobei das Anschlußstück einstückig mit dem Handhabenteil oder als separates, an dem Handhabenteil festlegbares Adapterstück ausgebildet sein kann.

Das Verrasten von Schaft und Hülse kann dadurch erleichtert werden, daß der Schaft zumindest im Bereich des den größeren Außendurchmesser aufweisenden freien Endes radial verformbar ausgebildet ist. Ein sicherer Halt der Rastverbindung kann dabei dadurch sichergestellt werden, daß der Außendurchmesser des Schaftes im Bereich des freien Endes den Innendurchmesser der Hülse im Bereich des geringsten Innendurchmessers überschreitet, so daß zum Verrasten der Schaft zunächst aufgrund seiner radialen Verformbarkeit zusammengepreßt werden muß, bevor er sich einen sicheren Halt gewährend in der Hinterschneidung wieder entspannt.

Um dem Schaft die radiale Verformbarkeit zu ermöglichen, wird erfindungsgemäß vorgeschlagen, daß im Schaft zumindest im Bereich des den größeren Außendurchmesser aufweisenden freien Endes zumindest ein sich in Längsrichtung des Schaftes erstreckender Schlitz ausgebildet ist. Durch diesen Schlitz ist es möglich, den Schaft federnd zusammenzudrücken, um den Engpaß in der Hülse zu überwinden.

Das Verrasten und Lösen der beiden Bauteile kann materialschonend dadurch erleichtert werden, daß sowohl in Axialrichtung des Schaftes gesehen an beiden Enden des Bereichs mit dem größeren Außendurchmesser als auch in Axialrichtung der Hülse gesehen an beiden Enden des Bereichs mit dem geringsten Innendurchmesser Anlaufschrägen ausgebildet sind.

Schließlich wird mit der Erfindung vorgeschlagen, daß der Griffteil und/oder das Anschlußstück aus Kunststoff, insbesondere einem spritzgegossenen Kunststoff, bestehen. Die Ausbildung zumindest des Griffteils als Spritzgußteil aus Kunststoff stellt eine kostengünstige und fertigungstechnisch einfache Lösung dar.

Weitere Merkmale und Vorteile der Erfindung ergeben sich anhand der nachfolgenden Beschreibung der zugehörigen Zeichnung, in der ein Ausführungsbeispiel eines erfindungsgemäß ausgebildeten medizinischen Instruments beispielhaft dargestellt ist. In der Zeichnung zeigt:
- Fig. 1: eine schematische Seitenansicht eines passiven Resektoskops;
- Fig. 2: eine teilweise geschnittene Seitenansicht eines als Fingerring ausgebildeten Griffteils vor dem Verrasten mit einem Anschlußstück;
- Fig. 3: eine Ansicht gemäß Fig. 2, jedoch den Fingerring in der mit dem Anschlußstück verrasteten Stellung darstellend und
- Fig. 4: eine verkleinerte Draufsicht auf den Fingerring gemäß Fig. 2.

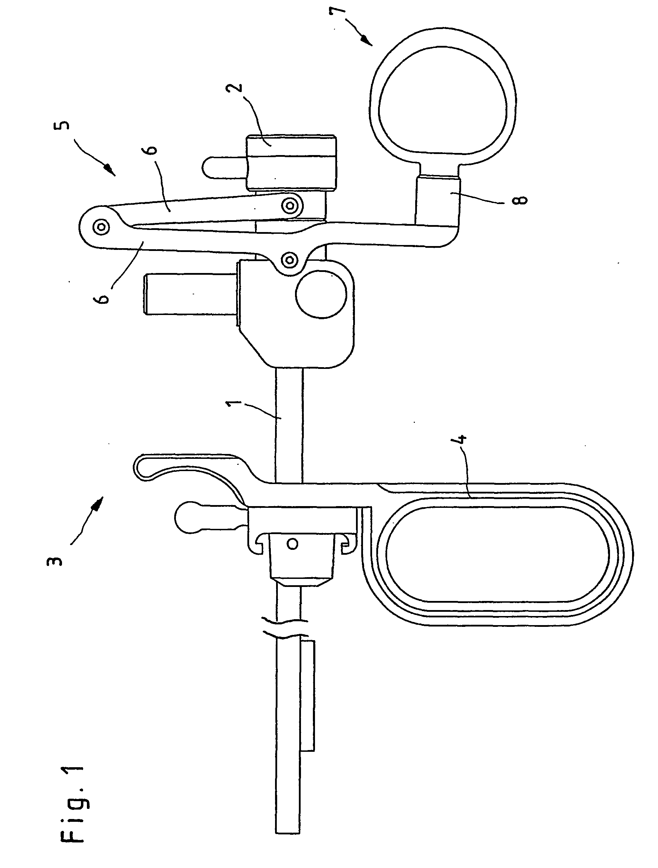

Bei dem in Fig. 1 schematisch dargestellten medizinischen Instrument handelt es sich um ein passives Resektoskop. Resektoskope werden bei bestimmten chirurgischen Eingriffen verwendet, um unter endoskopischer Sicht Gewebeteile zu entfernen. Das dargestellte Resektoskop besteht im wesentlichen aus einem hohlen Schaft 1, einem in dem Schaft 1 gelagerten Endoskop 2 und einer am proximalen Ende des Schaftes 1 angeordneten Handhabe 3, über die ein nicht dargestelltes Werkzeug parallel zum Schaft 1 geführt werden kann.

Wie aus Fig. 1 ersichtlich, besteht die Handhabe 3 aus einem mit dem Schaft 1 verbundenen starren Handhabenteil 4 und einem relativ zu dem starren Handhabenteil 4 bewegbaren Handhabenteil 5 zum Betätigen des Werkzeugs. Bei dem dargestellten Ausführungsbeispiel ist der bewegliche Handhabenteil 5 über einen Hebelmechanismus aus mehreren gelenkig miteinander verbundenen Hebeln 6 einseitig am Endoskop 2 festgelegt. Bei aktiven Resektoskopen ist dieser Aufbau genau umgekehrt. Dort ist der bewegliche Handhabenteil 5 auf der distalen Seite angeordnet. Zum exakten Führen des beweglichen Handhabenteils 5 und somit auch des Werkzeugs ist der bewegliche Handhabenteil 5 mit einem Griffteil 7, insbesondere einem Fingerring, versehen, über den der Operateur das Werkzeug gezielt und gefühlvoll unter endoskopischer Sichtkontrolle führen kann. Um dem Operateur eine möglichst große Bewegungsfreiheit zu ermöglichen, ist der Griffteil 7 um die Längsachse frei drehbar an einem Handhabenteil 4, 5 festgelegt, nämlich proximal am beweglichen Handhabenteil 5 bei einem passiven Resektoskop und distal am starren Handhabenteil 4 bei einem aktiven Resektoskop.

Um weiterhin das Resektoskop bestmöglich an den jeweiligen Operateur anpassen zu können, ist der Griffteil 7 lösbar und damit austauschbar mit dem, im dargestellten Ausführungsbeispiel beweglichen Handhabenteil 5 verbunden, so daß unterschiedlich große und unterschiedlich geformte Griffteile 7 angeschlossen werden können, ohne das Resektoskop wechseln zu müssen.

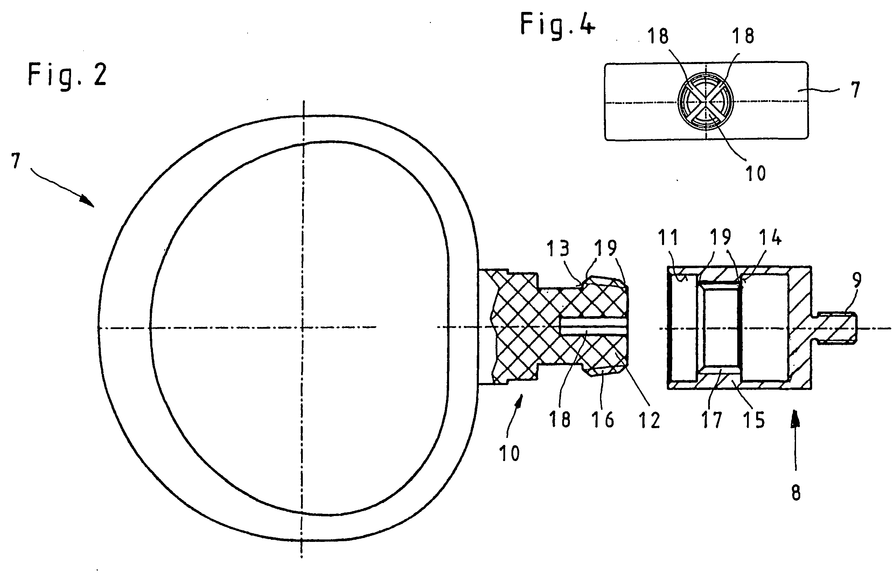

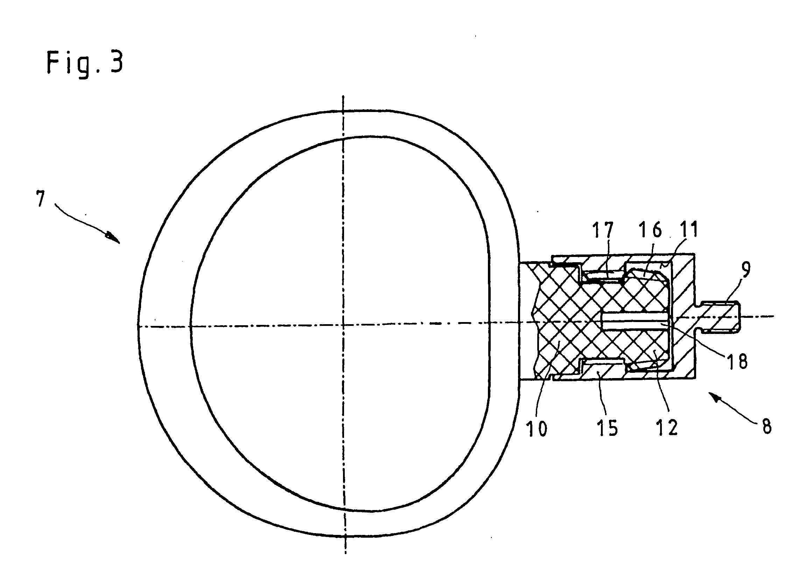

Die Art der Verbindung zwischen dem Handhabenteil 5 einerseits und dem Griffteil 7 andererseits ist detailliert den Abbildungen Fig. 2 und 3 zu entnehmen. Wie aus den Abbildungen ersichtlich, handelt es sich bei der dargestellten Verbindung um eine Rastverbindung, mit der der Griffteil 7 an einem Anschlußstück 8 des bewglichen Griffteils 5 festlegbar ist. Das Anschlußstück 8 kann dabei entweder einstükkig mit dem Handhabenteil 5 oder aber als separates Adapterstück ausgebildet sein, welches seinerseits beispielsweise über ein Gewinde 9 am Handhabenteil 5 festlegbar ist, wie dies die Abbildungen Fig. 2 und 3 zeigen. Selbstverständlich ist es auch möglich, den Griffteil 7 über die Rastverbindung direkt an einem Handhabenteil 4, 5 festzulegen.

Die eigentliche Rastverbindung besteht aus einem an einem der miteinander zu verbindenden Bauteile 7 oder 8 ausgebildeten Schaft 10 und einer an dem jeweils anderen Bauteil 8 oder 7 ausgebildeten Hülse 11 zur Aufnahme des Schaftes 10.

Bei dem dargestellten Ausführungsbeispiel ist der Schaft 10 am Griffteil 7 und die Hülse 11 in dem Anschlußstück 8 ausgebildet.

Wie aus Fig. 2 ersichtlich, weist der am Griffteil 7 ausgebildete Schaft 10 Bereiche unterschiedlichen Außendurchmessers auf. Zur Ausbildung der Rastverbindung überragt der Außendurchmesser des Schaftes 10 im Bereich 12 des freien Endes den Kerndurchmesser im mittleren Bereich des Schaftes 10, so daß hier durch den Kalibersprung eine Hinterschneidung 13 ausgebildet wird. Die zur Aufnahme des Schaftes 10 dienende, im Anschlußstück 8 ausgebildete Hülse 11 weist eine der Außenkontur des Schaftes 10 entsprechende Innenform mit einem eine Hinterschneidung 14 bildenden Bereich mit größerem Innendurchmesser auf, der zur Aufnahme des den größeren Außendurchmesser aufweisenden Bereich 12 am freien Ende des Schaftes 10 dient. In Richtung zur Hülsenöffnung schließt sich an die Hinterschneidung 14 ein Bereich 15 mit einem geringeren Innendurchmesser an, der in der verrasteten Stellung im Bereich der Hinterschneidung 13 des Schaftes 10 angeordnet ist, wie dies die Abbildung Fig. 3 zeigt.

Bei dem dargestellten Ausführungsbeispiel ist der Schaft 10 im Bereich 12 mit dem größeren Außendurchmesser mit einem Außengewinde 16 versehen, welches bevorzugt konisch ausgebildet ist und mit einem Innengewinde 17 korrespondiert, das in der Hülse 11 im Bereich 15 mit dem geringsten Innendurchmesser ausgebildet ist.

Um eine sichere Rastverbindung zu schaffen, die auch axial wirkenden Zugkräften standhält, ist der Schaft 10 im Bereich 12 des größten Außendurchmessers mit einem geringen Übermaß gegenüber dem Bereich 15 der Hülse 11 mit dem geringsten Innendurchmesser ausgebildet. Damit trotz des Übermaßes des Schaftes 10 die Rastverbindung geschlossen und wieder geöffnet werden kann, ist der Schaft 10 zumindest im Bereich 12 des größten Außendurchmessers in Radialrichtung kompressibel ausgebildet. Zu diesem Zweck sind in dem dargestellten Schaft 10 zwei sich rechtwinklig kreuzende Schlitze 18 ausgebildet, wie dies insbesondere der Abbildung Fig. 4 zu entnehmen ist.

Das Anschließen und Lösen eines Griffteils 7 an das Anschlußstück 8 eines Handhabenteils 4, 5 der Handhabe 3 geschieht wie folgt:

Ausgehend von der in Fig. 2 dargestellten Ausgangslage, in der das Anschlußstück 8 als separates Adapterstück ausgebildet ist, ist es zunächst notwendig, das Anschlußstück 8 mit einem Handhabenteil 4, 5 der Handhabe 3 zu verbinden. Im dargestellten Fall ist es der bewegliche Handhabenteil 5. Zu diesem Zweck wird das Anschlußstück 8 über das Gewinde 9 in ein entsprechendes Innengewinde des beweglichen Handhabenteils 5 eingeschraubt.

Anschließend wird der am Griffteil 7 angeformte Schaft 10 in die Hülse 11 des Anschlußstücks 8 eingeschoben, bis der Bereich 12 mit dem größten Außendurchmesser am freien Ende des Schaftes 10 an den Bereich 15 der Hülse 11 mit dem geringsten Innendurchmesser anstößt. Durch Aufbringen einer axialen Druckkraft wird der aufgrund der Schlitze 18 radial verformbare Schaft 10 soweit zusammengedrückt, daß der Auslauf des am Schaft 10 ausgebildeten Außengewindes 16 in das in der Hülse 11 ausgebildete Innengewinde 17 eingreifen kann.

Das Einführen des Außengewindes 16 in das Innengewinde 17 wird dadurch erleichtert, daß die Bereiche 12 und 15 mit dem größten Außendurchmesser des Schaftes 10 und dem geringsten Innendurchmesser der Hülse 11 jeweils beidseitig mit Anlaufschrägen 19 versehen sind.

Das Einschrauben des Schaftes 10 in die Hülse 11 wird fortgesetzt, bis der mit dem Außengewinde 16 versehene Bereich 12 des Schaftes 10 den mit dem Innengewinde 17 versehenen Bereich 15 der Hülse 11 vollständig passiert hat und der Bereich 12 des Schaftes 10 mit dem größten Außendurchmesser in den Bereich der Hülse 11 eingetreten ist, in dem die Hinterschneidung 14 ausgebildet ist. Diese die verrastete Stellung darstellende Position ist der Abbildung Fig. 3 zu entnehmen.

Sobald das freie Ende des Schaftes 10 in den Bereich der Hinterschneidung 14 eintritt, entspannt sich der radial verformte Schaft 10 wieder, so daß der Schaft 10 aufgrund seines Übermaßes im Bereich des freien Endes nunmehr in Axialrichtung zugfest in der Rastverbindung gehalten wird. In dieser Stellung ist der Schaft 10 frei drehbar in der Hülse 11 gelagert, wobei der Bereich 12 mit dem größten Außendurchmesser des Schaftes 10 nur an der im Bereich 15 der Hülse 11 ausgebildeten Fase anliegt, so daß beim Verdrehen des Schaftes 10 bzw. des Griffteils 7 kaum materialverschleißende Reibungskräfte auftreten.

Das Demontieren des Griffteils 7 erfolgt in umgekehrter Reihenfolge. Durch das Aufbringen einer axialen Zugkraft wird der Schaft 10 soweit radial verformt, bis das Außengewinde 16 in das Innengewinde 17 eingreifen kann. Anschließend werden die beiden Bauteile Griffteil 7 und Anschlußstück 8 auseinander geschraubt, bis sie wieder die in Fig. 2 dargestellte Position einnehmen.

Insgesamt zeichnet sich diese Rastverbindung zwischen Griffteil 7 einerseits und Handhabe 3 andererseits dadurch aus, daß die Bauteile fertigungstechnisch einfach und kostengünstig, beispielsweise als Kunststoff-Spritzgußteile herstellbar sind und die Rastverbindung die Festlegung des Griffteils 7 in axialer Richtung bei gleichzeitiger Verdrehbarkeit um die Längsachse ermöglicht.

### Bezugszeichenliste

- 1: Schaft
- 2: Endoskop
- 3: Handhabe
- 4: starrer Handhabenteil
- 5: beweglicher Handhabenteil
- 6: Hebel
- 7: Griffteil
- 8: Anschlußstück
- 9: Gewinde
- 10: Schaft
- 11: Hülse
- 12: Bereich des größten Außendurchmessers
- 13: Hinterschneidung
- 14: Hinterschneidung
- 15: Bereich des geringsten Innendurchmessers
- 16: Außengewinde
- 17: Innengewinde
- 18: Schlitz
- 19: Anlaufschräge

## Patentansprüche

1. Medizinisches Instrument, insbesondere Resektoskop, mit einer Handhabe (3), an der an einem Handhabenteil (4, 5) der Handhabe (3) drehbar gelagert mindestens ein Griffteil (7), insbesondere ein Fingerring, über eine Rastverbindung lösbar festgelegt ist, wobei die Rastverbindung aus einem an einem der miteinander zu verbindenden Bauteile (4, 5, 7) ausgebildeten Schaft (10) und einer am jeweils anderen Bauteil (7, 4, 5) ausgebildeten Hülse (11) zur Aufnahme des Schaftes (10) besteht, wobei der Schaft (10) Bereiche unterschiedlichen Außendurchmessers aufweist und der Außendurchmesser des Schaftes (10) im Bereich des freien Endes des Schaftes (10) eine Hinterschneidung (13) bildend den Kerndurchmesser des Schaftes (10) übersteigt und der Innendurchmesser der Hülse (11) entsprechend einen eine Hinterschneidung (14) bildenden und zur Aufnahme des freien Endes des Schaftes (10) dienenden Bereich mit einem größeren Innendurchmesser und einen sich in Richtung der Hülsenöffnung erstreckenden Bereich (15) mit einem geringeren Innendurchmesser aufweist,
**dadurch gekennzeichnet,**
**daß** der Schaft (10) im Bereich (12) des den größeren Außendurchmesser aufweisenden freien Endes mit einem Außengewinde (16) versehen ist und in der Hülse (11) im Bereich (15) des geringsten Innendurchmessers ein Innengewinde (17) ausgebildet ist.

2. Medizinisches Instrument nach Anspruch 1, **dadurch gekennzeichnet, daß** der Griffteil über ein Anschlußstück (8) an einem Handhabenteil (4, 5) festlegbar ist.

3. Medizinisches Instrument nach Anspruch 1 oder 2, **dadurch gekennzeichnet, daß** der Schaft (10) zumindest im Bereich (12) des den größeren Außendurchmesser aufweisenden freien Endes radial verformbar ausgebildet ist.

4. Medizinisches Instrument nach mindestens einem der Ansprüche 1 bis 3, **dadurch gekennzeichnet, daß** der Außendurchmesser des Schaftes (10) im Bereich (12) des freien Endes den Innendurchmesser der Hülse (11) im Bereich (15) des geringsten Innendurchmessers überschreitet.

5. Medizinisches Instrument nach Anspruch 4, **dadurch gekennzeichnet, daß** im Schaft (10) zumindest im Bereich (12) des den größeren Außendurchmesser aufweisenden freien Endes zumindest ein sich in Längsrichtung des Schaftes (10) erstreckender Schlitz (18) ausgebildet ist.

6. Medizinisches Instrument nach mindestens einem der Ansprüche 1 bis 5, **dadurch gekennzeichnet, daß** sowohl in Axialrichtung des Schaftes (10) gesehen an beiden Enden des Bereichs (12) mit dem größeren Außendurchmesser als auch in Axialrichtung der Hülse (11) gesehen an beiden Enden des Bereichs (15) mit dem geringsten Innendurchmesser Anlaufschrägen (19) ausgebildet sind.

7. Medizinisches Instrument nach mindestens einem der Ansprüche 1 bis 6, **dadurch gekennzeichnet, daß** der Griffteil (7) und/oder das Anschlußstück (8) aus Kunststoff, insbesondere einem spritzgegossenen Kunststoff, bestehen.

## Claims

1. Medical instrument, especially a resectoscope, with a handle (3) on which at least one grip part (7), in particular a finger ring, mounted rotatably on one handle part (4, 5) of the handle (3) is secured releasably via a catch connection, the catch connection being composed of a shaft (10) formed on one of the components (4, 5, 7) that are to be connected to one another, and of a sleeve (11) which is formed on the respective other component (7, 4, 5) and receives the shaft (10), the shaft (10) having areas of different external diameter, and the external diameter of the shaft (10), forming an undercut (13) in the area of the free end of the shaft (10), exceeds the core diameter of the shaft (10), and the internal diameter of the sleeve (11) correspondingly has an area of greater internal diameter forming an undercut (14) and serving to receive the free end of the shaft (10), and an area (15) extending in the direction of the sleeve opening and having a smaller internal diameter, **characterized in that** the shaft (10), in the area (12) of the free end with the greater external diameter, is provided with an outer thread (16), and an inner thread (17) is formed in the area (15) of the smallest internal diameter in the sleeve (11).

2. Medical instrument according to Claim 1, **characterized in that** the grip part can be secured on a handle part (4, 5) via a connector piece (8).

3. Medical instrument according to Claim 1 or 2, **characterized in that** the shaft (10) is radially deformable, at least in the area (12) of the free end having the greater external diameter.

4. Medical instrument according to at least one of Claims 1 to 3, **characterized in that** the external diameter of the shaft (10) in the area (12) of the free end exceeds the internal diameter of the sleeve (11) in the area (15) of the smallest internal diameter.

5. Medical instrument according to Claim 4, **characterized in that** in the shaft (10), at least in the area (12) of the free end having the greater external diameter, at least one slit (18) is formed extending in the longitudinal direction of the shaft (10).

6. Medical instrument according to at least one of Claims 1 to 5, **characterized in that** run-up bevels (19) are formed, seen both in the axial direction of the shaft (10), at both ends of the area (12) with the greater external diameter, and also in the axial direction of the sleeve (11), at both ends of the area (15) with the smallest internal diameter.

7. Medical instrument according to at least one of Claims 1 to 6, **characterized in that** the grip part (7) and/or the connector piece (8) are made of plastic, in particular an injection-moulded plastic.

## Revendications

1. Instrument médical, en particulier résectoscope, comportant une manette (3), sur laquelle au moins une partie de préhension (7), en particulier un anneau de préhension, montée pivotante sur une partie (4, 5) de la manette (3), est fixée de manière amovible par l'intermédiaire d'un assemblage encliqueté, ledit assemblage encliqueté étant formé par une queue (10), réalisée sur l'une des parties (4, 5, 7) à assembler entre elles, et par une douille (11), réalisée sur l'autre partie (7, 4, 5) correspondante et destinée à recevoir la queue (10), la queue (10) comportant des zones de diamètre extérieur différent et le diamètre extérieur de la queue (10), dans la zone de l'extrémité libre de la queue (10) formant une contre-dépouille (13), est supérieur au diamètre de l'âme de la queue (10) et, de manière correspondante, le diamètre intérieur de la douille (11) comporte une zone avec un plus grand diamètre intérieur, qui forme une contre-dépouille (14) et est destinée à recevoir l'extrémité libre de la queue (10), et une zone (15) avec un plus petit diamètre intérieur qui s'étend vers l'ouverture de la douille,
**caractérisé en ce que** la queue (10), dans la zone (12) de l'extrémité libre avec le plus grand diamètre extérieur, est munie d'un filet (16) et **en ce qu'**un taraudage (17) est réalisé dans la douille (11) dans la zone (15) avec le plus petit diamètre intérieur.

2. Instrument médical selon la revendication 1, **caractérisé en ce que** la partie de préhension (7) peut être fixée sur la partie de manette (4, 5) au moyen d'un raccord (8).

3. Instrument médical selon la revendication 1 ou 2, **caractérisé en ce que** la queue (10) peut se déformer dans le sens radial au moins dans la zone (12) de l'extrémité libre avec le plus grand diamètre extérieur.

4. Instrument médical selon l'une quelconque des revendications 1 à 3, **caractérisé en ce que** le diamètre extérieur de la queue (10) dans la zone (12) de l'extrémité libre est plus grand que le diamètre intérieur de la douille (11) dans la zone (15) avec le plus petit diamètre intérieur.

5. Instrument médical selon la revendication 4, **caractérisé en ce que** dans la queue (10), au moins dans la zone (12) de l'extrémité libre avec le plus grand diamètre extérieur, est réalisée au moins une fente (18) orientée dans le sens longitudinal de la queue (10).

6. Instrument médical selon l'une quelconque des revendications 1 à 5, **caractérisé en ce que** des rampes d'introduction (19) sont réalisées dans la direction axiale de la queue (10) au niveau des deux extrémités de la zone (12) avec le plus grand diamètre extérieur, de même que dans la direction axiale de la douille (11) au niveau des deux extrémités de la zone (15) avec le plus petit diamètre intérieur.

7. Instrument médical selon l'une quelconque des revendications 1 à 6, **caractérisé en ce que** la partie de préhension (7) et/ou le raccord (8) sont réalisés en matière plastique, en particulier dans une matière plastique moulée par injection.
